# EUROPEAN PATENT APPLICATION

(11) **EP 2 168 568 A1**
(43) Date of publication of application: **31.03.2010**
(21) Application number: 07790201.3
(22) Date of filing: 22.06.2007
(51) Int. Cl.: A61K 8/64, A61K 8/24, A61K 8/36, A61K 8/81

(54) **SKIN COSMETIC**

(71) Applicant: Shiseido Company, Limited, Chuo-ku Tokyo 104-8010 (JP)
(72) Inventor: OMURA, Takayuki, Yokohama-shi, Kanagawa 224-8558 (JP)
(74) Representative: Merkle, Gebhard
(86) International application number: PCT/JP2007/000676
(87) International publication number: WO 2007/125657

(57) **Abstract**

A skin cosmetic comprising the following ingredients (A), (B), and (C), and also satisfying the condition (D):
(A) β -alanyl-L-histidine and/or its salt
(B) A thickener consisting of a microgel obtained by radical polymerization of water soluble ethylene-type unsaturated monomers dissolved in the dispersion phase in a composition having an organic solvent or an oil component as the dispersion medium and water as the dispersion phase.
(C) Inorganic acid and/or organic acid
(D) The pH of the skin cosmetic at 25 °C is 5.0 or higher and lower than 8.0.

The present invention provides a skin cosmetic having superior sensation during use and stability and also maximally manifesting the various effects of ingredient (A), which is β - alanyl-L-histidine and/or its salt, by preparing a mildly acidic skin cosmetic by characteristically using ingredient (A) and ingredient (B), which is a thickener composed of a specific microgel chosen from many cosmetic thickeners, and adding ingredient (C), which is an inorganic acid and/or organic acid.

## Description

### TECHNICAL FIELD

The present invention relates to a skin cosmetic containing β-alanyl-L-histidine and/or its salt (trivial name: carnosine, basic dipeptide), known as a drug having effects on skin damage/UV induced immune suppression, anti-wrinkling, parakeratosis suppression, and pore reduction, and yet also superior in terms of usability and stability.

### BACKGROUND ART

β-alanyl-L-histidine, also called L-carnosine, is known to be widely distributed in skeletal muscles of various animals (Non-Patent document 1). Physiological functions are not clear, but anti-oxidation actions and metal chelating actions have been reported.
Patent applications have been filed for β-alanyl-L-histidine as a cosmetic inside and outside of Japan and applications as skin cosmetics that have superior beautifying effects such as anti-wrinkling (Patent Document 1), skin activation, revitalization, and whitening have been described (Patent Document 2).
Also, patent applications for medicinal drugs focusing on its pharmacological actions have been filed as well [for example, heat burn cure (Patent document 3), eczematous skin disease cure (Patent Document 4), senile essential pruritus cure (Patent Document 5), anti-tumor agent (Patent Document 6), anti-ulcer agent (Patent Document 7), anti-tumor agent (Patent Document 8), hepatopathy prevention cure (Patent Document 9), immunomodulator agent (Patent Document 10), and osteoporosis prevention agent (Patent Document 11).
Also, a PCT application pertaining to utilization of β-alanyl-L-histidine in skin cosmetics or similar applications has been published (Patent Document 12).

Patent Document 1: EP 2003-254760 bulletin
Patent Document 2: Japanese Patent Laid-Open H2-221213 bulletin
Patent Document 3: Japanese Patent Publication S60-45161 bulletin
Patent Document 4: Japanese Patent Publication H1-45451 bulletin
Patent Document 5: Japanese Patent Publication H1-49130 bulletin
Patent Document 6: Japanese Patent Publication H3-12045 bulletin
Patent Document 7: Japanese Patent Publication H3-5367 bulletin
Patent Document 8: Japanese Patent Publication H3-78368 bulletin
Patent Document 9: Japanese Patent Publication H4-62299 bulletin
Patent Document 10: Japanese Patent Publication H4-81966 bulletin
Patent Document 11: Japanese Patent Publication H7-17505 bulletin
Patent Document: PCT/AU89/00422 bulletin
Non-Patent Document 1: Dictionary of Biochemistry (3rd edition), Tokyo Kagaku Dojin, 1998, p318

As described above, β-alanyl-L-histidine and/or its salt (INCI designation: carnosine, basic dipeptide) is known as a drug that has an effect on skin damage/UV induced immune suppression, anti-wrinkling, parakeratosis suppression, and pore reduction.
However, since it is made of histidine, which is a basic amino acid, an aqueous solution having a sufficient concentration of it to have the aforementioned effects becomes basic (pH 8 or more) and therefore is not preferable as a mildly acidic skin cosmetic. Therefore, in the past the proper effects of β-alanyl-L-histidine and/or its salt could not be fully utilized in skin cosmetics.

On the other hand, a thickener is generally used when preparing a skin cosmetic. However, when a pH adjustment agent such as citric acid or lactic acid was added to a formulation that was basic due to the contained β-alanyl-L-histidine and/or its salt in order to shift the pH to the mildly acidic region, the basicity of β-alanyl-L-histidine and/or its salt and the acidity of the acid acted like salt to the thickener, which caused stability problems for those thickeners lacking salt resistance.

On the other hand, if succinoglycan, xanthan gum and such, which were known to be thickeners with salt resistance, are used, then stability was superior but there were problems in terms of the sensation during use such as freshness and stickiness.

### DISCLOSURE OF INVENTION

### TECHNICAL PROBLEM

In view of the aforementioned problems, the inventors conducted earnest research and discovered that a skin cosmetic having a sufficient thickening effect, no stickiness, a fresh sensation during use, and superior stability can be obtained by preparing a skin cosmetic by combining (A) β-alanyl-L-histidine and/or its salt, (B) a thickener composed of a microgel composed of a specific synthetic polymer electrolyte, and (C) an inorganic acid and/or organic acid to prepare (D) a skin cosmetic whose pH at 25 °C is 5.0 or more and lower than 8.0, thus completing the present invention.

The object of the present invention is to provide a skin cosmetic having a superior sensation during use and stability and also maximally manifesting the various effects of β-alanyl-L-histidine and/or its salt by preparing a mildly acidic skin cosmetic by characteristically using β-alanyl-L-histidine and/or its salt and a thickener composed of a specific microgel chosen from many cosmetic thickeners and adding an inorganic acid and/or organic acid.
That is, the object is to provide a useful mildly acidic skin cosmetic, superior in terms of sensations during use and stability, which can never be obtained by using thickeners generally used in skin cosmetics such as carboxyvinyl polymer and synthetic polymer electrolyte polymer gels prepared with the homogeneous polymerization method or the reverse phase suspension polymerization method.

### TECHNICAL SOLUTION

That is, the present invention provides a skin cosmetic comprising the following ingredients (A), (B), and (C), and also satisfying the condition (D) :
(A) β-alanyl-L-histidine and/or its salt
(B) A thickener consisting of a microgel obtained by radical polymerization of water soluble ethylene-type unsaturated monomers dissolved in the dispersion phase in a composition having an organic solvent or an oil component as the dispersion medium and water as the dispersion phase.
(C) Inorganic acid and/or organic acid
(D) The pH of the skin cosmetic at 25 °C is 5.0 or higher and lower than 8.0.

Also, the present invention provides the aforementioned skin cosmetic wherein the microgel in said (B) is a microgel obtained by radical polymerization of a composition having an organic solvent or an oil component as the dispersion medium and water as the dispersion phase under conditions in which a single phase microemulsion or fine W/O emulsion is formed by using a surfactant.

Furthermore, the present invention provides the aforementioned skin cosmetic wherein the apparent viscosity of a 0.5 wt% water dispersion of the microgel of said (B) at 25 °C is 10,000 Pa-s or more at a shear rate of 1.0 s⁻¹.

Also, the present invention provides the aforementioned skin cosmetic wherein the apparent viscosity of a 0.5 wt% ethanol dispersion of the microgel of said (B) at 25 °C is 5,000 Pa-s or more at a shear rate of 1.0 s⁻¹.

Furthermore, the present invention provides the aforementioned skin cosmetic wherein the dynamic elastic modulus of a water or ethanol dispersion having 0.5 wt% of the microgel of said (B) at 25 °C satisfies a relationship G' (stored elastic modulus) > G" (loss elastic modulus) at a strain of 1% or less and a frequency range of 0.01-10 Hz.

Furthermore, the present invention provides the aforementioned skin cosmetic wherein the water soluble ethylene-type unsaturated monomer of said (B) is a dialkylacrylamide represented by general formula (1) and a ionic acrylamide derivative represented by general formula (2) or (3).

### General formula (1)

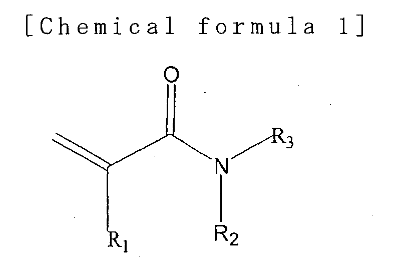

(R₁ denotes a H or methyl group; R₂ and R₃, independent of each other, denote a methyl, ethyl, propyl, or isopropyl group.)

### General formula (2)

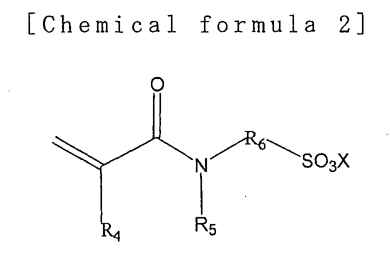

(R₄ and R₅, independent of each other, denote a H or methyl group, R₆ denotes a straight chain or branched alkyl group having 1-6 carbon atoms, and X denotes a metal ion, NH₃, or an amine compound.)

### General formula (3)

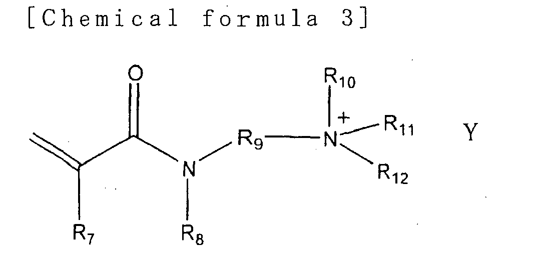

(R₇ denotes a H or methyl group, R₈ denotes a H or straight chain or branched alkyl group having 1-6 carbon atoms, R₉ denotes a straight chain or branched alkyl group having 1-6 carbon atoms, R₁₀, R₁₁, and R₁₂ denote a methyl group or ethyl group, and Y denotes an anionic counter ion.)

Furthermore, the present invention provides the aforementioned skin cosmetic wherein the blend ratio of said ingredient (A) is 0.1-3.0 wt% of the total amount of the skin cosmetic, the blend ratio of said ingredient (B) is 0.1-5.0 wt% of the total amount of the skin cosmetic, and the blend ratio of said ingredient (C) is 0.01-1.0 wt% of the total amount of the skin cosmetic.

Also, the present invention provides the aforementioned skin cosmetic wherein the inorganic acid and/or organic acid of said ingredient (C) is one, two, or more chosen from a group consisting of phosphoric acid, lactic acid, and citric acid.

### BEST MODE FOR CARRYING OUT THE INVENTION

The present invention is described in detail below.

### <Ingredient (A)>

Ingredient (A), β-alanyl-L-histidine, is commercially available as a reagent and can be easily procured. It can also be synthesized by using a prior art method.

In the present invention, β-alanyl-L-histidine and/or its salt is used.

Selection of this salt is not limited in particular; examples of the inorganic salt include chlorides, sulfates, phosphates, hydrobromide, sodium salts, potassium salts, magnesium salts, calcium salts, and ammonium salts. Examples of the organic salt include acetates, lactates, maleates, fumarates, tartrates, citrates, methanesulfonate, p-toluenesulfonates, triethanolamine salts, diethanolamine salts, and amino acid salts.

β-alanyl-L-histidine can be turned into an inorganic salt or an organic salt by using a prior art method.

Examples of commercially available β-alanyl-L-histidine include Dragosine 2/060700 (from Symrise) and Oripeptide CNS (from Orient Stars LLC). Examples of a commercially available β-alanyl-L-histidine salt include Carcinine (2HCl) (from Exsymol).

The blend ratio of ingredient (A) is preferably 0.1-3.0 wt% of the total amount of the skin cosmetic. If it is less than 0.1 wt%, then β-alanyl-L-histidine and/or its salt cannot sufficiently manifest its effects. Also, blending more than 3.0 wt% would not increase the effects.

### <Ingredient (B) >

A thickener consisting of the microgel of ingredient (B) is a synthetic polymer microgel obtained by radical polymerization of water soluble ethylene-type unsaturated monomers dissolved in the dispersion phase in a composition having an organic solvent or an oil component as the dispersion medium and water as the dispersion phase.
That is, a polymer microgel manufactured by the polymerization method generally called the reverse emulsion polymerization method is used as a thickener; its polymerization method and mechanical properties are different from those of a thickener consisting of a synthetic polymer obtained by a homogeneous polymerization system disclosed in, for example, Japanese Patent Laid-Open No. 2001-114641 bulletin.

A microgel is synthetic polymer electrolyte fine particles manufactured by means of the reverse phase microemulsion polymerization method. The thickener consisting of the microgel used in the present invention swells in water, ethanol, or a water/ethanol mixed solution to provide a highly viscous solution that appears homogeneous to the naked eye.

The polymerization system for the microgel used as the thickener in the present invention is different from the homogeneous polymerization system for preparing conventional thickeners that are synthetic polymer. For example, the synthetic polymer from the homogeneous polymerization system disclosed in Japanese Patent Laid-Open No. 2001-114641 bulletin is not the microgel used in the present invention; this synthetic polymer has to be crushed to powder after polymerization before being added to a cosmetic. Also, the synthetic polymer gel stands out and may cause an appearance problem.

In contrast, the microgel used in the present invention is polymerized in an inhomogeneous polymerization system. The obtained synthetic polymer is a fine polymer gel, or microgel; it does not have to be crushed into powder before being added to a cosmetic, it exhibits a superior thickening effect and a superior sensation during use, and it gives a good appearance to the cosmetic.

An example of the reverse phase emulsion polymerization method for polymers, described in Japanese Patent No. 1911623 bulletin, manufactures a water-swelling polymer using acrylic acid by means of reverse phase polymerization and uses it as a thickener; this, however, is different from the microgel used in the present invention that improves the shortcomings of carboxyvinyl polymers widely used today.

Furthermore, Japanese Patent Laid-Open No. H9-12613 bulletin discloses a method to use the reverse phase emulsion polymerization method to manufacture water-absorbing microgel particles and make them into a certain size so they are suitable for diapers and menstrual sanitary products; this technology, however, cannot be used for cosmetic thickeners and is completely different from the microgel used in the present invention.

The thickener used in the present invention is prepared by using the reverse phase emulsification polymerization method (Japanese Patent Laid-Open 2004-114641 bulletin). That is, this thickener is manufactured by radical polymerization of water soluble ethylene-type unsaturated monomers dissolved in the dispersion phase in a composition having an organic solvent or an oil component as the dispersion medium and water as the dispersion phase. The polymerized microgel is rinsed and dried, but there is no need for crushing.
It is preferable to manufacture the thickener consisting of a microgel by using a surfactant whose hydrophilicity/lipophilicity balance (HLB) is specifically selected so that the reverse emulsion polymerization system forms a single phase microemulsion or fine W/O emulsion.

A single phase microemulsion is a state in which the oil phase and the water phase coexist in a thermodynamically stable manner and the surface tension between the oil and water is at a minimum. A fine W/0 emulsion is a state in which the oil and the water are present as a fine W/O emulsion in a thermodynamically unstable but kinetically stable manner. Generally, the particle size of the inner water phase of a fine W/O emulsion is several tens to 100 nm. These states are determined solely by the system composition and the temperature, and are not affected by the mechanical agitation conditions, for example.

The composition that makes up the polymerization system consists of the dispersion medium (outer phase) consisting of an organic solvent or oil component that does not mix with water and the dispersion phase (inner phase) consisting of water. Examples of preferable organic solvents include alkanes such as pentane, hexane, heptane, octane, nonane, decane, and undecane; cycloalkanes such as cyclopentane, cyclohexane, cycloheptane, and cyclooctane; and aromatic and cyclic hydrocarbons such as benzene, toluene, xylene, decaline, and naphthalene.
Examples of preferable oil components include non-polar oil components such as paraffin oil.

The water soluble ethylene type unsaturated monomer is dissolved in water, which is the dispersion phase, and then mixed with the organic solvent or oil component, which is the dispersion medium; after the temperature is raised to the prescribed temperature, the polymerization initiator is added to the water phase to carry out the polymerization.
In general, with heterogeneous polymerization methods, it is known that the physical properties of the polymer that is manufactured are different depending on the agitation conditions during the polymerization. This is because the emulsion system is not thermodynamically stable and as a result the shape and the size of the emulsified particles change depending on the agitation conditions. In the present invention, it was found that these problems can be avoided by carrying out the polymerization in the thermodynamically stable single phase microemulsion region or the metastable fine W/O emulsion region near the single phase region. Specifically, it has become possible to obtain a microgel with a good thickening effect by polymerizing the polymer in a fine water phase (water droplets) by adjusting the composition of the polymerization system (organic solvent type, HLB of the surfactant) in such a manner that the single phase microemulsion region or the fine W/0 emulsion region appears near the optimum polymerization temperature for the polymerization initiator for an ordinary thermal polymerization or redox polymerization.

On the contrary, in the case of the polymer thickener from a conventional suspension polymerization (for example, the method described in Japanese Patent Laid-Open No. 2001-1146641) bulletin, the particle size of water drops during polymerization is difficult to control and a microgel of good quality is difficult to obtain.

For the water soluble ethylene type unsaturated monomer, joint use of a nonionic monomer and an ionic monomer (anionic monomer or cationic monomer) is preferable.
For the nonionic monomer, the dialkylacrylamide represented by the following general formula (1) is preferable. For the ionic monomer, the anionic acrylamide derivative represented by the following general formula (2) or the cationic acrylamide derivative represented by the following general formula (3) is preferable.

### General formula (1)

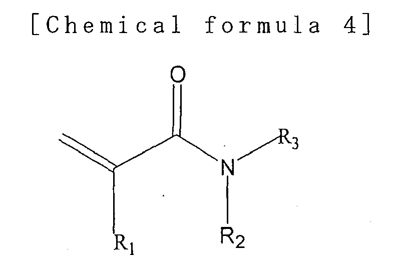

(R₁ denotes a H or methyl group; R₂ and R₃, independent of each other, denote a methyl, ethyl, propyl, or isopropyl group.)

### General formula (2)

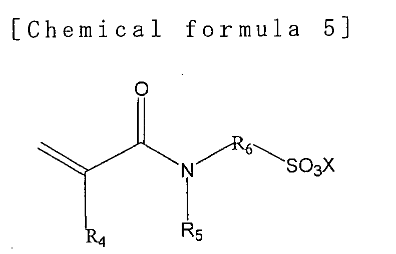

(R₄ and R₅, independent of each other, denote a H or methyl group, R₆ denotes a straight chain or branched alkyl group having 1-6 carbon atoms, and X denotes a metal ion, NH₃, or an amine compound. For example, the metal ion is Li, Na, or K, and the amine compound is triethanolamine or triisopropanolamine.)

### General formula (3)

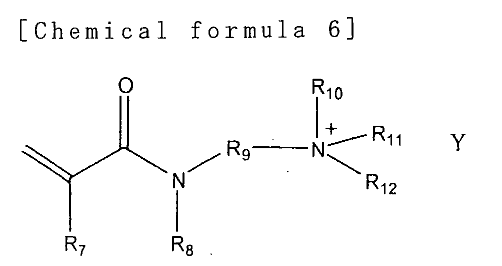

(R₇ denotes a H or methyl group, R₈ denotes a H or straight chain or branched alkyl group having 1-6 carbon atoms, R₉ denotes a straight chain or branched alkyl group having 1-6 carbon atoms, R₁₀, R₁₁, and R₁₂ denote a methyl group or ethyl group, and Y denotes an anionic counter ion, such as negative counter ions including Cl and Br.)

Particularly preferable dialkylacrylamides are dimethylacrylamide and diethylacrylamide.
Particularly preferable ionic acrylamide derivatives are 2-acrylamide-2-methylpropanesulfonic acid and its salts.
A particularly preferable cationic acrylamide derivative is N,N-dimethylaminopropylacrylamidemethyl chloride.

The monomer composition ratio of the nonionic monomer and the ionic monomer in the polymerization system (feed ratio of the polymerization system) is selected based on the monomer composition ratio of the target microgel. The monomer composition ratio of the microgel and the feed ratio into the polymerization system are about the same. The feed ratio of the nonionic monomer and the ionic monomer in the polymerization system (molar ratio) for copolymerization is usually in the range of Nonionic monomer : Ionic monomer = 0.5 : 9.5 to 9.5 : 0.5, preferably 1 : 9 to 9 : 1, more preferably 7 : 3 to 9 : 1. The optimum ratio is Nonionic monomer : Ionic monomer = 8 : 2.

The aforementioned water soluble ethylene type unsaturated monomer is then chosen at will and the thickener of the present invention is polymerized. A particularly preferable thickener is a dipolymer microgel copolymerized from monomers of dimethylacrylamide and 2-acrylamide-2-methylpropanesulfonic acid, used as the water soluble ethylene type unsaturated monomer. In this case, without requiring a cross-linking monomer, a thickener that exhibits a superior thickening effect and sensation during use can be obtained by self cross-linking. A cross-linking monomer can be used; a cross-linking monomer represented by general formula (4) is preferable, and methylenebisacrylamide is particularly preferable.

### General formula (4)

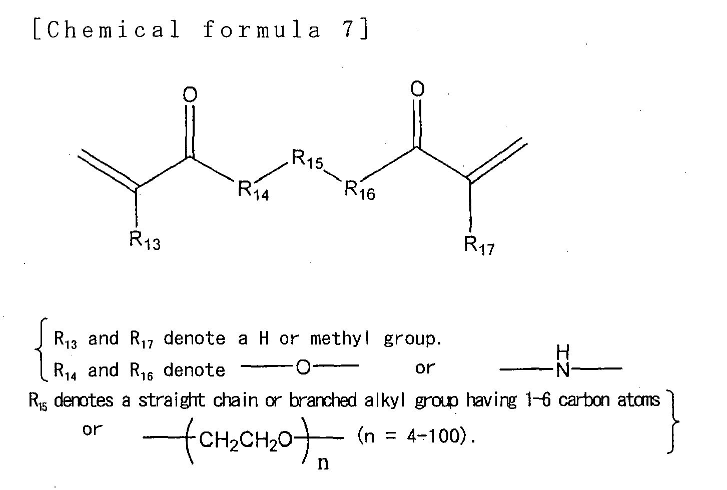

In order to dissolve the water soluble ethylene type unsaturated monomer in the dispersion phase and polymerize a microgel preferable for the present invention, it is necessary to select the optimum outer phase oil component or organic solvent and the optimum surfactant. The inventors discovered that a microgel having preferable rheological properties as a thickener can be obtained by creating a condition wherein a single phase microemulsion or fine W/0 emulsion is formed at a usual thermal radical polymerization temperature by adjusting the hydrophilicity/lipophilicity balance (HLB) of the nonionic surfactant by preparing a phase diagram and thus preparing the polymerization system composition in such a way that the clouding point is at the temperature suitable for the thermal radical polymerization.

Preferable surfactants include polyoxyethylenecetyl ether, polyoxyethyleneoleyl ether, polyoxyethylenestearyl ether, polyoxyethylenenonylphenyl ether, polyoxyethylenelauryl ether, polyoxyethylenehexyldecyl ether, polyoxyethyleneisostearyl ether, polyoxyethyleneoctyldodecyl ether, polyoxyethylenebehenyl ether, polyoxyethylenecholesteryl ether, polyoxyethylene hydrogenated castor oil, sorbitan fatty acid ester, mono-fatty acid glycerin, tri-fatty acid glycerin, polyglycerin fatty acid ester, polyoxyethyleneglycerin isostearate, polyoxyethyleneglycerin monostearate, polyoxyethyleneglyceryl distearate, and polyoxyethyleneglyceryl tristearate.
These surfactants can be combined to adjust the HLB to the desired level and then added to the polymerization system.

Also, in the case of the microgel obtained by copolymerizing a dialkylacrylamide and an acrylamide type ionic monomer, a spontaneous cross-linking reaction develops and a chemically self-cross-linked microgel can be obtained without having to copolymerize with a multifunctional cross-linking monomer as a third ingredient, thus providing a particularly preferable thickener for the present invention.

Although the third ingredient, the multifunctional cross-linking monomer, is not required, the microgel used in the present invention can still be synthesized if such a monomer is added for copolymerization. For the multifunctional cross-linking monomer, the monomers represented by general formula (6) are preferable; one, two, or more of the monomers represented by general formula (6) can be used for cross-linking. It is essential that these cross-linking monomers can effectively have a cross-linking structure in the polymerization system of the dialkylacrylamide and the ionic acrylamide derivative.

Examples of the cross-linking monomer include ethylene glycol diacrylate, ethylene glycol dimethacrylate, polyoxyethylene diacrylate, polyoxyethylene dimethacrylate, diethylene glycol dimethacrylate, trimethylolpropane triacrylate, N,N'-methylenebisacrylamide, N,N'-ethylenebisacrylamide, triallyl cyanurate, and pentaerythrithol dimethacrylate; one, two, or more chosen from these can be used. In the present invention, the use of N,N'-methylenebisacrylamide is particularly preferable.

In the copolymer that is the thickener used in the present invention, the content molar ratio of the 2-acrylamide-2-methylpropanesulfonic acid unit and the dialkylacrylamide unit is usually 2-acrylamide-2-methylpropanesulfonic acid unit : dialkylacrylamide unit = 0.5 : 9.5 to 9.5 : 0.5, preferably 1 : 9 to 9 : 1, and more preferably 3 : 7 to 1 : 9. The optimum ratio is 2-acrylamide-2-methylpropanesulfonic acid unit : dialkylacrylamide unit = 2 : 8. The viscosity of the thickener of the present invention results from the extension of the molecular chains due to the electrostatic repulsion of the sulfonyl group, which is a strongly dissociating group, and the spontaneous cross-linking reaction of dialkylacrylamide or the cross-linked structure formed by the cross-linking monomer; if the content of the 2-acrylamide-2-methylpropanesulfonic acid unit or its salt is less than 5 mole % compared with the dialkylacrylamide unit, then a sufficient viscosity may not be obtained because a sufficient extension of the molecular chains does not occur.

The blend ratio of the cross-linking monomer is preferably 0.0001-2.0 mole % of the total moles of the 2-acrylamide-2-methylpropanesulfonic acid or its salt and the dialkylacrylamide. If it is less than 0.0001 mole %, then the obtained thickener may not exhibit the effect of cross-linking. If more than 2 mole % is used for preparation, a sufficient thickening effect may not be achieved because the cross-link density is too high and the microgel cannot swell enough.

The weight average molecular weight of the microgel used in the present invention is 100,000-5,000,000 (PEG equivalent, measured with the GPC); it is adjusted according to the desired viscosity of the thickener.

The microgel obtained by the aforementioned polymerization method has all the rheological properties listed in (1)-(3) below. A thickener consisting of this microgel is obtained by the manufacturing method according to the aforementioned polymerization method and used preferably as a thickener.
(1) The apparent viscosity of the water dispersion having 0.5% (mass percentage) of the microgel in water is 10,000 mPa · s or higher at a shear rate of 1.0 s⁻¹.
(2) The apparent viscosity of the ethanol dispersion having 0.5% (mass percentage) of the microgel is 5000 mPa · s or higher at a shear rate of 1.0 s⁻¹.
(3) The dynamic elastic modulus of the water or ethanol dispersion having 0.5% (mass percentage) of it satisfies the relationship G' > G" at a strain of 1% or less and a frequency range of 0.01-10 Hz.

The apparent viscosity of the ethanol or water dispersion having the microgel is the viscosity measured with a cone/plate rheometer (MCR-300 from Paar Rhysica) at 25 °C and a shear rate of 1 s⁻¹.
The dynamic elastic modulus here means the stored elastic modulus (G') and the loss elastic modulus (G") measured at a strain of 1% or less and a frequency range of 0.1-10 Hz with the aforementioned measurement apparatus at a temperature of 25 °C.

Following the polymerization, the microgel to be used in the present invention can be isolated in a powder form after a precipitation/purification process. The microgel thus isolated in a powder form is easily dispersed in water, ethanol, or a water/ethanol mixed solvent and quickly swells and functions as a thickener.
Also, by choosing a strongly acidic monomer (a monomer containing a sulfonic acid residue, for example) for the ionic monomer to be copolymerized into the microgel, even an acidic formulation can be thickened, which was not possible with conventional carboxyvinyl polymers.

The skin cosmetic of the present invention is prepared by blending the aforementioned microgel as a thickener into the skin cosmetic base agent. The blend ratio of the thickener is determined according to the target skin cosmetic; there is no particular limitation. From the usability point of view, a preferable blend ratio is 0.01-10% (mass percentage), and more preferably 0.1-5% (mass percentage).

### <Ingredient (C) >

The inorganic and/or organic acid of ingredient (C) is described below. The inorganic and/or organic acid used in the present invention is used as a pH adjustment agent. Examples include phosphoric acid, lactic acid, and citric acid; one, two, or more of these are freely selected. When a skin cosmetic is an oil-in-water type emulsified composition or uses a water based base agent, the pH of the total composition is adjusted to 5.0 or higher and lower than 8.0. If the pH is lower than 5.0, then it is not preferable for a skin cosmetic because of too much acidity. A pH higher than 8.0 is also not preferable for a skin cosmetic because of too much basicity.

The present invention adjusts the pH of the skin cosmetic, which is raised higher than 8.0 by the action of ingredient (A), which comprises a basic amino acid, down to the range most preferable for a skin cosmetic by using ingredient (C), i. e. the inorganic and/or organic acid. This pH range is condition (D) (The pH of the skin cosmetic at 25 °C is 5.0 or higher and lower than 8.0).

When β-alanyl-L-histidine and/or its salt is added and then acid such as citric acid is added to adjust the pH of the skin cosmetic at 5.0 or higher and lower than 8.0, the pH-increasing action of β-alanyl-L-histidine and/or its salt and the pH-decreasing action of the acid combined have a 'salt' like action on thickeners that use electric charge repulsion, such as carboxyvinyl polymer and polyacrylamide, which causes a reduction in the viscosity and separation and such, leading to a stability problem.
On the other hand, nonionic thickeners such as xanthan gum and hydroxyethyl cellulose don't have such a stability problem; however, there still are usability problems such as poor absorption into the skin, lack of freshness, and stickiness.
However, the thickener ingredient (B) used in the present invention makes it possible to obtain a skin cosmetic that is free of the problem of the thickening effect instability, absorbs well into the skin, gives sensations of freshness and permeation, is free of stickiness, provides good usability even when β-alanyl-L-histidine and/or its salt and inorganic and/or organic acid are both added and the pH is adjusted to 5.0 or higher and lower than 8.0, which is a preferable range for skin cosmetics.

The present invention, by containing the aforementioned ingredients (A)-(C) and satisfying condition (D), provides a skin cosmetic that absorbs well into the skin, gives sensations of freshness and permeation, is free of stickiness, and thus gives superior sensations during use and yet exhibits superior stability over time despite the fact that it contains ingredient (A), which is known as a drug that has an effect on skin damage/UV induced immune suppression, anti-wrinkling, parakeratosis suppression, and pore reduction.

In addition to the aforementioned essential ingredients, other ingredients used in cosmetics can be blended as necessary in the skin cosmetic of the present invention; examples of such ingredients include powder ingredients, liquid fats and oils, solid fats and oils, waxes, hydrocarbons, higher fatty acids, higher alcohols, esters, silicones, anionic surfactants, cationic surfactants, ampholytic surfactants, nonionic surfactants, humectants, water-soluble polymers, thickeners, coating agents, ultraviolet absorbents, sequestering agents, lower alcohols, polyhydric alcohols, sugars, amino acids, organic amines, polymer emulsions, pH adjusting agents, skin nutrients, vitamins, antioxidants, antioxidation assistants, perfumes, and water; and the endermic liniment can be prepared for the target formulation with a conventional method. Specific ingredients which can be blended in are listed below. The skin cosmetic of the present invention can be prepared by blending the aforementioned essential ingredients and any one, two or more of the following ingredients.

Examples of the powder ingredients include inorganic powders (for example, talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, tungstic acid metal salt, magnesium, silica, zeolite, barium sulfate, firing calcium sulfate (calcined gypsum), calcium phosphate, fluorine-apatite, hydroxy apatite, ceramic powder, metallic soaps (for example, myristic acid zinc, calcium palmitate, and aluminum stearate), and boron nitride); organic powders (for example, polyamide resin powder (nylon powder), polyethylene powder, poly-methyl methacrylate powder, polystyrene powder, powders of the copolymer resin of styrene and acrylic acid, benzoguanamine resin powder, polytetrafluoroethylene powder, and cellulose powder); inorganic white pigments (for example, titanium dioxide and zinc oxide); inorganic red pigments (for example, iron oxide (red iron oxide) and iron titanate); inorganic brown pigments (for example, γ-iron oxide); inorganic yellow pigments (for example, yellow iron oxide and loess); inorganic black pigments (for example, black iron oxide and low oxides of titanium); inorganic purple pigments (for example, manganese violet, cobalt violet); inorganic green pigments (for example, chromium oxide, chromium hydroxide, and cobalt titanate); inorganic blue pigments (for example, ultramarine blue and Berlin blue); pearl pigment (for example, titania coated mica, titania coated bismuth oxychloride, titania coated talc, coloration titania coated mica, bismuth oxychloride, fish scale flakes); metal powder pigments (for example, aluminium powder, copper powder); organic pigments such as Zr, barium or aluminium rake (for example, organic pigments such as red 201, red 202, red 204, red 205, red 220, red 226, red 228, red 405, orange 203, orange 204, yellow 205, yellow 401 and blue 404, as well as red 3, red 104, red 106, red 227, red 230, red 401, red 505, orange 205, yellow 4, yellow 5, yellow 202, yellow 203, green 3 and blue 1; and natural colors (for example, chlorophyll and β-carotene).

Examples of the liquid fats and oils include avocado oil, tsubaki oil, turtle fatty acid, macademia nut oil, corn oil, mink oil, olive oil, rapeseed oil, egg yolk oil, sesame oil, persic oil, wheat germ oil, sasanqua oil, castor oil, linseed oil, safflower oil, cotton seed oil, perilla oil, soybean oil, peanut oil, tea seed oil, Japanese nutmeg oil, rice bran oil, Chinese gimlet oil, Japanese gimlet oil, jojoba oil, germ oil, and triglycerin.

Examples of the solid fats and oils include cacao butter, coconut oil, hydrogenated coconut oil, palm oil, palm kernel oil, Japanese core wax nucleus oil, hydrogenated oil, Japanese core wax, and hydrogenated castor oil.

Examples of the waxes include beeswax, candelilla wax, cotton wax, carnauba wax, bayberry wax, tree wax, whale wax, montan wax, bran wax, lanolin, kapok wax, lanolin acetate, liquid lanolin, sugar cane wax, lanolin fatty acid isopropyl ester, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol, and POE hydrogenated lanolin ethyl alcohol ether.

Examples of the hydrocarbon oils include liquid petrolatum, ozocerite, squalane, pristane, paraffin, ceresin, squalene, petrolatum, and microcrystallin wax.

Examples of the higher fatty acids include lauric acid, myristic acid, palmitic acid, stearic acid, behenic acid, oleic acid, undecylenic acid, tall oil fatty acid, isostearic acid, linolic acid, linoleic acid, eicosapentaenoic acid (EPA), and docosahexaenoic acid (DHA).

Examples of the higher alcohols include straight chain alcohols (for example, lauryl alcohol, cetyl alcohol, stearyl alcohol, behenyl alcohol, myristyl alcohol, oleyl alcohol, and cetostearyl alcohol) and branched chain ethyl alcohols (for example, mono s.tearyl glycerin ether (batyl alcohol), 2-decyltetradecynol, lanolin alcohol, cholesterol, phytosterol, hexyl dodecanol, iso stearyl alcohol, and octyl dodecanol).

Examples of the ester oils include isopropyl myristate, cetyl octanoate, octyl dodecyl myristate, isopropyl palmitate, butyl stearate, hexyl laurate, myristil myristate, decyl oleate, dimethyl hexyl decyl octanoate, cetyl lactate, myristil lactate, lanolin acetate, iso cetyl stearate, iso cetyl isostearate, cholesteryl 12-hydroxystearate, di-2-ethylene glycol ethylhexanoate, dipentaerythritol fatty acid ester, n-alkylene glycol monoisostearate, neopentyl glycol dicaprate, diisostearyl malate, glyceryl di-2-heptylundecanoate, trimethylolpropane tri-2-ethyl hexanoate, trimethylolpropane triisostearate, tetra-2-pentaerythritol ethylhexanoate, glycerin tri2-ethylhexanoate, glyceryl trioctanoate, glycerin triisopalmitate, trimethylolpropane triisostearate, cetyl 2-ethyl hexanoate, 2-ethylhexyl palmitate, glycerin trimyristate, tri-2-heptyl undecanoic acid glyceride, methyl castor oil fatty acid, oleyl oleate, aceto glyceride, 2-heptylundecyl palmitate, diisobutyl adipate, 2-octyldodecyl N-lauroyl-L-glutamate, di-2-heptylundecyl adipate, ethyl laurate, di2-ethylhexyl sebacate, 2-hexyldecyl myristate, 2-hexyldecyl palmitate, 2-hexyldecyl adipate, diisopropyl sebacate, 2-ethylhexyl succinate, and triethyl citrate.

Examples of the silicone oils include chain polysiloxanes (for example, dimethylpolysiloxane, methylphenyl polysiloxane, and diphenyl polysiloxane); ring polysiloxanes (for example, octamethylcyclotetrasiloxane, decamethyl cyclopenta siloxane, and dodecamethyl cyclohexa siloxane), silicone resins forming a three-dimensional network structure, silicone rubbers, and various modified polysiloxanes (amino-modified polysiloxane, polyether-modified polysiloxane, alkyl-modified polysiloxane, and fluorine-modified polysiloxane).

Examples of the anionic surfactants include fatty acid soaps (for example, sodium laurate and sodium palmitate); higher alkyl sulfuric ester salts (for example, sodium lauryl sulfate and potassium laurylsulfate); alkylether sulfuric ester salts (for example, POE-triethanolamine laurylsulfate and sodium POE-lauryl sulfate); N-acyl sarcosinic acids (for example, sodium N-lauroyl sarcosinate); higher fatty acid amide sulfonic acid salts (for example, sodium N-myristoyl N-methyl taurate, sodium cocoyl methyl taurate, and sodium laurylmethyl taurate); phosphoric ester salts (for example, sodium POE-oleyl ether phosphate and POE stearyl ether phosphoric acid); sulfosuccinates (for example sodium di-2-ethylhexylsulfosuccinate, sodium monolauroyl monoethanol amide polyoxyethylene sulfosuccinate, and sodium lauryl polypropylene glycol sulfosuccinate); alkyl benzene sulfonates (for example, sodium linear dodecyl benzene sulfonate, triethanolamine linear dodecyl benzene sulfonate, linear dodecyl benzene sulfonic acid); higher fatty acid ester sulfates (for example, hydrogenated coconut oil aliphatic acid glycerin sodium sulfate); N-acyl glutamates (for example, mono sodium N-lauroylglu-tamate, disodium N-stearoylglutamate, and sodium N-myristoyl-L-glutamate); sulfated oils (for example, turkey red oil); POE-alkylether carboxylic acid; POE-alkylarylether carboxylate; α-olefin sulfonate; higher fatty acid ester sulfonates; sec-alcohol sulfates; higher fatty acid alkyl amide sulfates; sodium lauroyl monoethanolamine succinates; ditriethanolamine N-palmitoylaspartate; and sodium caseinate.

Examples of the cationic surfactants include alkyl-trimethylammonium salts (for example, stearyltrimethyl ammonium chloride and lauryltrimethyl ammonium chloride) alkylpyridinium salts (for example, cetylpyridinium chloride), distearyldimethylammonium chloride dialkyldimethylammonium salt; poly (N,N'-dimethyl3-methylene piperidinium) chloride; alkyl quaternary ammonium salts; alkyl dimethylbenzyl ammonium salts; alkyl isoquinolinium salts; dialkylmorpholine salts; POE alkyl amines; alkyl amine salts; polyamine fatty acid derivatives; amylalcohol fatty acid derivatives; benzalkonium chloride; and benzethonium chloride.

Examples of the ampholytic surfactants include: imidazoline type ampholytic surfactants (for example, 2-undecyl-N, N, N-(hydroxyethyl carboxymethyl)2-imidazoline sodium salt and 2-coco y12-imidazolinium hydroxidel-carboxyethyloxy 2 sodium salt); and betaine type surtactants (for example, 2-heptadecyl-n-carboxymethyl-n-hydroxyethyl imidazolinium betaine, lauryldimethylaminoacetic acid betaine, alkyl betaine, amide betaine, and sulfobetaine).

Examples of the hydrophilic nonionic surface active agents include: polyglycerin fatty acid esters such as hexaglyceryl monolaurate (HLB 14.5), hexaglyceryl monomyristate (HLB 11), hexyglyceryl monostearate (HLB 9.0), hexaglyceryl monooleate (HLB 9.0), decaglyceryl monolaurate ((HLB 15.5), decaglyceryl monomyristate (HLB 14.0), decaglyceryl monostearate (HLB 12.0), decaglyceryl monoisostearate (HLB 12.0), decaglyceryl monooleate (HLB 12.0), decaglyceryl distearate (HLB 9.5), and decaglyceryl diisostearate (HLB 10.0); <0)
polyoxyethylene glycerin fatty acid esters such as polyoxyethylene (hereafter abbreviated as POE) glyceryl monostearate (5) (HLB 9.5), POE (15) glyceryl monostearate (HLB 13.5), POE (5) glyceryl monooleate (HLB 9.5), and POE (15) glyceryl monooleate (HLB 14.5);p
polyoxyethylene sorbitan fatty acid esters such as POE (20) sorbitan monococoate (HLB 16.9), POE (20) sorbitan monopalmitate (HLB 15.6), POE (20) sorbitan monostearate (HLB 14.9), POE (6) sorbitan monostearate (HLB 9.5), POE (20) sorbitan tristearate (HLB 10.5), POE (20) sorbitan monoisostearate (HLB 15.0), POE (20) sorbitan monooleate (HLB 15.0), POE (6) sorbitan monooleate (HLB 10.0), and POE (20) sorbitan trioleate (HLB 11.0);
polyoxyethylene sorbit fatty acid esters such as POE (6) sorbit monolaurate (HLB 15.5), POE (60) sorbit tetrastearate (HLB 13.0), POE (30) sorbit tetraoleate (HLB 11.5), POE (40) sorbit tetraoleate (HLB 12.5), and POE (60) sorbit tetraoleate (HLB 14.0);
polyoxyethylene lanolin/lanolin alcohol/beeswax derivatives such as POE (10) lanolin (HLB 12.0), POE (20) lanolin (HLB 13.0), POE (30) lanolin (HLB 15.0), POE (5) lanolin alcohol (HLB 12.5), POE (10) lanolin alcohol (HLB 15.5), POE (20) lanolin alcohol (HLB 16.0), POE (40) lanolin alcohol (HLB 17.0), and POE (20) sorbit beeswax (HLB 9.5);
polyoxyethylene castor oils/hydrogenated oils such as POE (20) castor oil (HLB 10.5), POE (40) castor oil (HLB 12.5), POE (50) castor oil (HLB 14.0), POE (60) castor oil (HLB 14.0), POE (20) hydrogenated castor oil (HLB 10.5), POE (30) hydrogenated castor oil (HLB 11.0), POE (40) hydrogenated castor oil (HLB 13.5), POE (60) hydrogenated castor oil (HLB 14.0), POE (80) hydrogenated castor oil (HLB 16.5), and POE (100) hydrogenated castor oil (HLB 16.5);
polyoxyethylene sterols/hydrogenated sterols such as POE (5) phytosterol (HLB 9.5), POE (10) phytosterol (HLB 12.5), POE (20) phytosterol (HLB 15.5), POE (30) phytosterol (HLB 18.0), POE (25) phytostanol (HLB 9.5), and POE (30) cholestanol (HLB 17.0);
polyoxyethylene alkyl ethers such as POE (2) lauryl ether (HLB 9.5), POE (4.2) lauryl ether (HLB 11. 5), POE (9) lauryl ether (HLB 14.5), POE (5.5) cetyl ether (HLB 10.5), POE (7) cetyl ether (HLB 11.5), POE (10) cetyl ether (HLB 13.5), POE (15) cetyl ether (HLB 15.5), POE (20) cetyl ether (HLB 17.0), POE (23) cetyl ether (HLB 18.0), POE (4) stearyl ether (HLB 9.0), POE (20) stearyl ether (HLB 18.0), POE (7) oleyl ether (HLB 10.5), POE (10) oleyl ether (HLB 14. 5), POE (15) oleyl ether (HLB 16.0), POE (20) oleyl ether (HLB 17.0), POE (50) oleyl ether (HLB 18.0), POE (10) behenyl ether (HLB 10.0), POE (20) behenyl ether (HLB 16.5), POE (30) behenyl ether (HLB 18.0), POE (2) (c12-15) alkyl ether (HLB 9.0), POE (4) (c12-15) alkyl ether (HLB 10.5), POE (10) (c12-15) alkyl ether (HLB15. 5), POE (5) secondary alkyl ether (HLB 10.5), POE (7) secondary alkyl ether (HLB 12.0), POE (9) alkyl ether (HLB 13.5), and POE (12) alkyl ether (HLB 14.5);
polyoxyethylene polyoxypropylene alkyl ethers such as polyoxyethylene (hereafter abbreviated as POE) (1) polyoxypropylene (hereafter abbreviated as POP) (4) cetyl ether (HLB 9.5), POE (10) POP (4) cetyl ether (HLB 10.5), POE (20) POP (8) cetyl ether (HLB 12.5), POE (20) POP (6) decyltetradecyl ether (HLB 11.0), and POE (30) POP (6) decyltetradecyl ether (HLB 12.0);
polyethylene glycol fatty acid esters such as polyethylene glycol (hereafter abbreviated as PEG) (10) (HLB 12.5), PEG (10) monostearate (HLB 11.0), PEG (25) monostearate (HLB 15.0), PEG (40) monostearate (HLB 17.5), PEG (45) monostearate (HLB 18.0), PEG (55) monostearate (HLB 18.0), PEG (10) monooleate (HLB 11.0), PEG distearate (HLB 16.5), and PEG diisostearate (HLB 9.5);
and polyoxyethylene glyceryl isostearates such as PEG (8) glyceryl isostearate (HLB 10.0), PEG (10) glyceryl isostearate (HLB 10.0), PEG (15) glyceryl isostearate (HLB 12.0), PEG (20) glyceryl isostearate (HLB 13.0), PEG (25) glyceryl isostearate (HLB 14.0), PEG (30) glyceryl isostearate (HLB 15.0), PEG (40) glyceryl isostearate (HLB 15.0), PEG (50) glyceryl isostearate (HLB 16.0), and PEG (60) glyceryl isostearate (HLB 16.0).

Examples of the lipophilic surfactant include POE (2) stearyl ether (HLB 4.0), self emulsified propylene glycol monostearate (HLB 4.0), glyceryl myristate (HLB 3.5), glyceryl monostearate (HLB 4.0), self emulsified glyceryl monostearate (HLB 4.0), glyceryl monoisostearate (HLB 4.0), glyceryl monooleate (HLB 2.5), hexaglyceryl tristearate (HLB 2.5), decaglyceryl pentastearate (HLB 3.5), decaglyceryl pontaisostearate (HLB 3.5), decaglyceryl pentaoleate (HLB 3.5), sorbitan monostearate (HLB 4.7), sorbitan tristearate (HLB 2.1), sorbitan monoisostearate (HLB 5.0), sorbitan sesquiisostearate (HLB 4.5), sorbitan monooleate (HLB 4.3), POE (6) sorbit hexastearate (HLB 3.0), POE (3) castor oil (HLB 3.0), PEG (2) monostearate (HLB 4.0), ethylene glycol monostearate (HLB 3.5), and PEG (2) stearate (HLB 4.5).

Examples of the humectant include polyethylene glycol, propylene glycol, glycerin, 1-butylene glycol, xylitol, sorbitol, maltitol, chondroitin sulfate, hyaluronic acid, mucoitin sulfuric acid, charonic acid, atelocollagen, cholesteryl12-hydroxy stearate, sodium lactate, bile salt, dl-pyrrolidone carboxylic acid salt, short chain soluble collagen, diglycerin (E0)P0 adduct, chestnut rose fruit extract, yarrow extract, and sweet clover extract.

Examples of the natural water-soluble polymer include: plant-type polymers (for example, gum arabic, gum tragacanth, galactan, guar gum, carob gum, karaya gum, carrageenan, pectin, agar, quince seed (Cydonia oblonga), algae colloids (brown algae extract), starches (rice, corn, potato, and wheat), and glycyrrhizic acid); microorganism-type polymers (for example, xanthan gum, dextran, succinoglucan, and pullulan); and animal-type polymers (for example, collagen, casein, albumin, and gelatin).

Examples of the semisynthetic water-soluble polymers include: starch-type polymers (for example, carboxymethyl starch and methylhydroxypropyl starch); cellulosic polymers (for example, methyl cellulose, ethyl cellulose, methylhydroxypropyl cellulose, hydroxyethyl cellulose, cellulose sodium sulfate, hydroxypropyl cellulose, carboxymetyl-cellulose, sodium carboxymethyl cellulose, crystal cellulose, and cellulose powder); and alginic acid-type polymers (for example, sodium alginate and propyleneglycol alginate).

Examples of the synthetic water-soluble polymers include: vinyl polymers (for example, polyvinyl alcohol, polyvinyl methyl ether, polyvinylpyrrolidone, carboxy vinyl polymer); polyoxyethylene-type polymers (for example, a copolymer of polyethylene glycol 20, 40, or 60 and polyoxyethylene polyoxypropylene); acrylic polymers (for example, sodium polyacrylate, polyethylacrylate, and polyacrylamide); polyethyleneimine; and cationic polymers.

Examples of the thickeners include: gum arabic, carrageenan, karaya gum, gum tragacanth, carob gum, quince seed (Cydonia oblonga), casein, dextrin, gelatin, sodium pectate, sodium arginate, methyl cellulose, ethyl cellulose, CMC, hydroxy ethyl cellulose, hydroxypropyl cellulose, PVA, PVM, PVP, sodium polyacrylate, carboxy vinyl polymer, locust bean gum, guar gum, tamarind gum, cellulose dialkyl dimethylammonium sulfate, xanthan gum, aluminum magnesium silicate, bentonite, hectorite, AlMg silicate (beagum), laponite, and silicic acid anhydride.

Examples of the ultraviolet absorbents include the following compounds.
(1) Benzoic acid-type ultraviolet absorbents Examples include paraminobenzoic acid (hereafter abbreviated as PABA), PABA monoglycerin ester, N, N-dipropoxy PABA ethyl ester, N, N-diethoxy PABA ethyl ester, N, N-dimethyl PABA ethyl ester, N, N-dimethyl PABA butyl ester, and N,N-dimethyl PABA ethyl ester.
(2) Anthranilic acid-type ultraviolet absorbents Examples include homo mentyl-N-acetyl anthranilate.
(3) Salicylic acid-type ultraviolet absorbents Examples include amyl salicylate, mentyl salicylate, homo mentyl salicylate, octyl salicylate, phenyl salicylate, benzyl salicylate, and p-isopropanol phenyl salicylate.
(4) Cinnamic acid-type ultraviolet absorbents Examples include octyl cinnamate, ethyl -4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate, 2-ethylhexyl-p-methoxy cinnamate, 2-ethoxyethyl-p-methoxy cinnamate, cyclohexyl-p-methoxy cinnamate, ethyl-α-cyano-β-phenyl cinnamate, 2-ethylhexyl-α-cyano-β-phenyl cinnamate, and glyceryl mono-2-ethyl hexanoyl-diparamethoxy cinnamate.
(5) Triazine-type ultraviolet absorbents Examples include bisresorsinyl triazine. More specifically, bis{[4-(2-ethylhexyloxy)-2-hydroxy]phenyl}-6-(4-methoxyphenyl)1,3,5-triazine, 2,4,6-tris{4-(2-ethylhexyloxycarbonyl)anilino}1,3,5-triazine, etc.
(6) Other ultraviolet absorbents Examples include 3-(4'-methylbenzylidene)-d,1-camphor, 3-benzylidene-d,1-camphor, 2-phenyl-5-methyl benzoxazole, 2,2'-hydroxy-5-methylphenyl benzotriazol, 2-(2'-hydroxy-5'-t-octylphenyl) benzotriazol, 2-(2'-hydroxy-5'-methylphenyl benzotriazol, dibenzaladine, dianisoylmethane, 4-methoxy-4'-t-butyl dibenzoyl-methane, and 5-(3,3-dimethyl-2-norbornylidene)-3-pentane-2-one. Also, pyridazine derivatives such as dimorpholinopyridazinone.

Examples of the sequestering agents include 1-hydroxy ethanel-diphosphonic acid, 1-hydroxy ethanel-diphosphonic acid tetrasodium salt, disodium edetate, trisodium edetate, tetrasodium edetate, sodium citrate, sodium polyphosphate, sodium metaphosphate, gluconic acid, phosphoric acid, citric acid, ascorbic acid, succinic acid, and trisodium ethylenediaminehydroxyethyl triacetate.

Examples of the lower alcohols include ethanol, propanol, isopropanol, isobutyl alcohol, and t-butyl alcohol.

Examples of the polyhydric alcohols include:
dihydric alcohols (for example, ethylene glycol, propylene glycol, trimethylene glycol, 1-butylene glycol, 2-butylene glycol, tetramethylene glycol, 2-butylene glycol, pentamethylene glycol, 2-butene-1,4-diol, hexylene glycol, and octylene glycol); trihydric alcohols (for example, glycerin and trimethylolpropane); tetrahydric alcohols (for example, pentaerythritol such as 12-hexanetriol);
pentahydric alcohols (for example, xylitol);
hexahydric alcohols (for example, sorbitol, mannitol); polyhydric alcohol polymers (for example, diethylene glycol, dipropylene glycol, triethylene glycol, polypropylene glycol, tetraethylene glycol, diglycerin, polyethylene glycol, triglycerin, tetraglycerin, and polyglycerin); dihydric alcohol alkylethers (for example, ethylene glycol monomethyl ether, ethylene glycol monoethyl ether, ethylene glycol monobutyl ether, ethylene glycol monophenyl ether, ethylene glycol monohexyl ether, ethylene glycol mono 2-methyl hexyl ether, ethylene glycol isoamyl ether, ethylene glycol benzyl ether, ethylene glycol isopropyl ether, ethylene glycol dimethylether, ethylene glycol diethyl ether, and ethylene glycol dibutyl ether); dihydric alcohol ether esters (for example, ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycolmonomethyl ether acetate, propylene glycol monoethyl ether acetate, propylene glycol monopropyl ether acetate, and propylene glycol monophenyl ether acetate);
glycerin mono alkyl ethers (for example, chimyl alcohol, selachyl alcohol, and batyl alcohol);
sugar alcohols (for example, sorbitol, maltitol, maltotriose, mannitol, sucrose, erythritol, glucose, fructose, starch amylolysis sugar, maltose, xylitose, and alcohol prepared by the reduction of starch amylolysis sugar); glysolid;
tetrahydro furfuryl alcohol; POE-tetrahydro furfuryl alcohol; POP-butyl ether; POP/POE-butyl ether; tripolyoxypropylene glycerin ether; POP-glycerin ether, POP-glycerin ether phosphoric acid; POP/POE-pentane erythritol ether, and polyglycerin.

Examples of the monosaccharides include: trioses (for example, D-glyceryl aldehyde and dihydroxyacetone); tetroses (for example, D-etythrose, D-erythrulose, D-threose, and erythritol); pentoses (for example, L-arabinose, D-xylose, L-lyxose, D-arabinose, D-ribose, D-ribulose, D-xylulose, and L-xylulose); hexoses (for example, D-glucose, D-talose, D-psicose, D-galactose, D-fructose, L-galactose, L-mannose, and D-tagatose); heptoses (for example, aldoheptose and heprose); octoses (for example, octurose); deoxysugars (for example, 2-deoxy-D-ribose, 6-deoxy-L-galactose, and 6-deoxy-L-mannose); amino sugars (for example, D-glucosamine, D-galactosamine, sialic acid, amino uronic acid, and muramic acid); and uronic acid (for example, D-glucuronic acid, D-mannuronic acid, L-guluronic acid, D-galacturonic acid, and L-iduronic acid).

Examples of the oligosaccharides include sucrose, umbelliferose, lactose, planteose, isolignoses, alpha, α-trehalose, raffinose, lignoses, umbilicine, stachyose and verbascose.

Examples of the polysaccharides include cellulose, quince seed, chondroitin sulfate, starch, galactan, dermatan sulfate, glycogen, gum arabic, heparan sulfate, hyaluronic acid, traganth gum, keratan sulfate, chondroitin, xanthan gum, mucoitin sulfuric acid, guar gum, dextran, kerato sulfate, locustbean gum, succinoglucane, and charonic acid.

Examples of amino acids include neutral amino acids (for example, threonine and cysteine) and basic amino acids (for example, hydroxylysine). Examples of the amino acid derivatives include sodium acyl sarcosinate (sodium N-lauroyl sarcosinate), acyl glutamate, sodium acyl β-alanine, glutathione, and pyrrolidone carboxylic acid.

Examples of the organic amines include monoethanolamine, diethanolamine, triethanolamine, morpholine, triisopropanolamine, 2-amino-2-methyl-1,3-propanediol, and 2-amino-2-methyl-1-propanol.

Examples of polymer emulsions include acrylic resin emulsions, ethyl polyacrylate emulsions, acryl resin liquids, polyacrylic alkyl ester emulsions, polyvinyl acetate resin emulsions, and natural rubber latex.

Examples of the pH adjustment agents include buffers such as lactic acid-sodium lactate, citric acid-sodium citrate, and succinic acid-sodium succinate.

Examples of vitamins include vitamin A, B1, B2, B6, C and E as well as their derivatives, pantothenic acid and its derivatives, and biotin.

Examples of the antioxidants include tocopherols, dibutyl hydroxytoluene, butyl hydroxyanisole, and gallic ester.

Examples of antioxidation assistants include phosphoric acid, citric acid, ascorbic acid, maleic acid, malonic acid, succinic acid, fumaric acid, cephalin, hexametaphosphate, phytic acid, and ethylene diamine tetraacetic acid.

Examples of other possible ingredients include antiseptics (methylparaben, ethylparaben, butylparaben, and phenoxyethanol); antiinflammatory agents (for example, glycyrrhizic acid derivatives, glycyrrhetinic acid derivatives, salicylic acid derivatives, hinokitiol, zinc oxide, and allantoin); whitening agents (for example, creeping saxifrage extract and arbutin); various extracts (for example, Phellodendri Cortex, goldthread, lithospermum root , Paeonia lactiflora, Swertia japonica, Birch, sage, loquat, carrot, aloe, Malva sylvestris, Iris, grape, Coix ma-yuen, sponge gourd, lily, saffron, Cnidium officinale, sheng jiang, Hypericum erectum, Ononis, garlic, Guinea pepper, chen pi, Ligusticum acutilobum, and seaweed), activators (royal jelly, photosensitive substances, and cholesterol derivatives); blood circulation promoting agents (for example, nonyl acid valenyl amide, nicotinic acid benzyl esters, nicotinic acid β -butoxy ethyl esters, capsaicin, gingeron, cantharis tincture, Ichthammol, tannic acid, α -borneol, tocopherol nicotinate, inositol hexanicotinate, cyclandelate, cinnarizine, tolazoline, acetylcholine, verapamil, cepharanthine, and γ -orizanol); anti-seborrhea agents (for example, sulfur and thiantol); and antiinflammatory agents (for example, tranexamic acid, thiotaurine, and hypotaurine).

The product form of the skin cosmetic of the present invention is not limited; it can be used for skin cosmetics such as lotions, emulsions, cream, packs, etc.

### EXAMPLES

The present invention is described below in specific detail through examples, but the present invention is not limited by these examples. The blend ratios in Examples are in wt% (mass-percentage) units unless specified otherwise.
First, the synthesis examples of the microgel used in the present invention are described. The microgel obtained from a synthesis example is a thickener of the present invention.

### Synthesis example 1

40 g of dialkylacrylamide (from Kohjin) and 9 g of 2-acrylamide-2-methylpropanesulfonic acid (from Sigma) are dissolved in 250 g of ion-exchanged water and the pH is adjusted to 7.0 with sodium hydroxide. 250 g of n-hexane, 8.2 g of polyoxyethylene (3) oleyl ether (EMALEX 503, made by Nihon Emulsion), and 16.4 g of polyoxyethylene (6) oleyl ether (EMALEX 506, made by Nihon Emulsion) are put into a 1,000 ml three-neck flask provided with a refluxing apparatus, mixed and dissolved, and then subjected to N₂ substitution. The monomer aqueous solution is added to this three-neck flask, and the temperature is raised to 65 °C-70 °C using an oil bath as stirring is carried out in an N₂ atmosphere. When the system temperature reaches 65 °C-70 °C, after confirming that the system has taken on a semitransparent microemulsion state, 2 g of ammonium persulfate is added to the polymerization system to start the polymerization. The temperature of the polymerization system is maintained at 65 °C-70 °C for three hours while stirring to obtain the microgel. After the completion of the polymerization, acetone is added to the microgel suspension to precipitate the microgel, followed by rinsing with acetone three times to remove the remaining monomers and the surfactant. The precipitate is filtered and then dried under reduced pressure to obtain the dried microgel in a white powder form.

### Synthesis example 2

35 g of dialkylacrylamide (from Kohjin) and 17.5 g of 2-acrylamide-2-methylpropanesulfonic acid (from Sigma) are dissolved in 260 g of ion-exchanged water and the pH is adjusted to 7.0 with sodium hydroxide. 260 g of n-hexane, 8.7 g of polyoxyethylene (3) oleyl ether (EMALEX 503, made by Nihon Emulsion), and 17.6 g of polyoxyethylene (6) oleyl ether (EMALEX 506, made by Nihon Emulsion) are put into a 1,000 ml three-neck flask provided with a refluxing apparatus, mixed and dissolved, and then subjected to N₂ substitution. The monomer aqueous solution is added to this three-neck flask, and the temperature is raised to 65 °C-70 °C using an oil bath as stirring is carried out in an N₂ atmosphere. When the system temperature reaches 65 °C-70 °C, after confirming that the system has taken on a semitransparent microemulsion state, 2 g of ammonium persulfate is added to the polymerization system to start the polymerization. The temperature of the polymerization system is maintained at 65 °C-70 °C for three hours while stirring to obtain the microgel. After the completion of the polymerization, acetone is added to the microgel suspension to precipitate the microgel, followed by rinsing with acetone three times to remove the remaining monomers and the surfactant. The precipitate is filtered and then dried under reduced pressure to obtain the dried microgel in a white powder form.

### Synthesis example 3

30 g of dialkylacrylamide (from Kohjin) and 26.7 g of 2-acrylamide-2-methylpropanesulfonic acid (from Sigma) are dissolved in 280 g of ion-exchanged water and the pH is adjusted to 7.0 with sodium hydroxide. 280 g of n-hexane, 9.4 g of polyoxyethylene (3) oleyl ether (EMALEX 503, made by Nihon Emulsion), and 19 g of polyoxyethylene (6) oleyl ether (EMALEX 506, made by Nihon Emulsion) are put into a 1,000 ml three-neck flask provided with a refluxing apparatus, mixed and dissolved, and then subjected to N₂ substitution. The monomer aqueous solution is added to this three-neck flask, and the temperature is raised to 65 °C-70 °C using an oil bath as stirring is carried out in an N₂ atmosphere. When the system temperature reaches 65 °C-70 °C, after confirming that the system has taken on a semitransparent microemulsion state, 2 g of ammonium persulfate is added to the polymerization system to start the polymerization. The temperature of the polymerization system is maintained at 65 °C-70 °C for three hours while stirring to obtain the microgel. After the completion of the polymerization, acetone is added to the microgel suspension to precipitate the microgel, followed by rinsing with acetone three times to remove the remaining monomers and the surfactant. The precipitate is filtered and then dried under reduced pressure to obtain the dried microgel in a white powder form.

### Synthesis example 4

35 g of dialkylacrylamide (from Kohjin), 17.5 g of 2-acrylamide-2-methylpropanesulfonic acid (from Sigma), and 7 mg of methylene bisacrylamide are dissolved in 260 g of ion-exchanged water and the pH is adjusted to 7.0 with sodium hydroxide. 260 g of n-hexane, 8.7 g of polyoxyethylene (3) oleyl ether (EMALEX 503, made by Nihon Emulsion), and 17.6 g of polyoxyethylene (6) oleyl ether (EMALEX 506, made by Nihon Emulsion) are put into a 1,000 ml three-neck flask provided with a refluxing apparatus, mixed and dissolved, and then subjected to N₂ substitution. The monomer aqueous solution is added to this three-neck flask, and the temperature is raised to 65 °C-70 °C using an oil bath as stirring is carried out in an N₂ atmosphere. When the system temperature reaches 65 °C-70 °C, after confirming that the system has taken on a semitransparent microemulsion state, 2 g of ammonium persulfate is added to the polymerization system to start the polymerization. The temperature of the polymerization system is maintained at 65 °C-70 °C for three hours while stirring to obtain the microgel. After the completion of the polymerization, acetone is added to the microgel suspension to precipitate the microgel, followed by rinsing with acetone three times to remove the remaining monomers and the surfactant. The precipitate is filtered and then dried under reduced pressure to obtain the dried microgel in a white powder form.

### Synthesis example 5

35 g of dialkylacrylamide (from Kohjin), 17.5 g of 2-acrylamide-2-methylpropanesulfonic acid (from Sigma), and 70 mg of methylene bisacrylamide are dissolved in 260 g of ion-exchanged water and the pH is adjusted to 7.0 with sodium hydroxide. 260 g of n-hexane, 8.7 g of polyoxyethylene (3) oleyl ether (EMALEX 503, made by Nihon Emulsion), and 17.6 g of polyoxyethylene (6) oleyl ether (EMALEX 506, made by Nihon Emulsion) are put into a 1,000 ml three-neck flask provided with a refluxing apparatus, mixed and dissolved, and then subjected to N₂ substitution. The monomer aqueous solution is added to this three-neck flask, and the temperature is raised to 65 °C-70 °C using an oil bath as stirring is carried out in an N₂ atmosphere. When the system temperature reaches 65 °C-70 °C, after confirming that the system has taken on a semitransparent microemulsion state, 2 g of ammonium persulfate is added to the polymerization system to start the polymerization. The temperature of the polymerization system is maintained at 65 °C-70 °C for three hours while stirring to obtain the microgel. After the completion of the polymerization, acetone is added to the microgel suspension to precipitate the microgel, followed by rinsing with acetone three times to remove the remaining monomers and the surfactant. The precipitate is filtered and then dried under reduced pressure to obtain the dried microgel in a white powder form.

### Synthesis example 6

35 g of dialkylacrylamide (from Kohjin) and 17.5 g of N,N-dimethylaminopropylacrylamidemethyl chloride (from Kohjin) are dissolved in 260 g of ion-exchanged water. 260 g of n-hexane, 8.7 g of polyoxyethylene (3) oleyl ether (EMALEX 503, made by Nihon Emulsion), and 17.6 g of polyoxyethylene (6) oleyl ether (EMALEX 506, made by Nihon Emulsion) are put into a 1,000 ml three-neck flask provided with a refluxing apparatus, mixed and dissolved, and then subjected to N₂ substitution. The monomer aqueous solution is added to this three-neck flask, and the temperature is raised to 65 °C-70 °C using an oil bath as stirring is carried out in an N₂ atmosphere. When the system temperature reaches 65 °C-70 °C, after confirming that the system has taken on a semitransparent microemulsion state, 2 g of ammonium persulfate is added to the polymerization system to start the polymerization. The temperature of the polymerization system is maintained at 65 °C-70 °C for three hours while stirring to obtain the microgel. After the completion of the polymerization, acetone is added to the microgel suspension to precipitate the microgel, followed by rinsing with acetone three times to remove the remaining monomers and the surfactant. The precipitate is filtered and then dried under reduced pressure to obtain the dried microgel in a white powder form.

### Synthesis example 7

35 g of dialkylacrylamide (from Kohjin), 17.5 g of N,N-dimethylaminopropylacrylamidemethyl chloride (from Kohjin), and 7 mg of methylenebisacrylamide are dissolved in 260 g of ion-exchanged water. 260 g of n-hexane, 8.7 g of polyoxyethylene (3) oleyl ether (EMALEX 503, made by Nihon Emulsion), and 17.6 g of polyoxyethylene (6) oleyl ether (EMALEX 506, made by Nihon Emulsion) are put into a 1,000 ml three-neck flask provided with a refluxing apparatus, mixed and dissolved, and then subjected to N₂ substitution. The monomer aqueous solution is added to this three-neck flask, and the temperature is raised to 65 °C-70 °C using an oil bath as stirring is carried out in an N₂ atmosphere. When the system temperature reaches 65 °C-70 °C, after confirming that the system has taken on a semitransparent microemulsion state, 2 g of ammonium persulfate is added to the polymerization system to start the polymerization. The temperature of the polymerization system is maintained at 65 °C-70 °C for three hours while stirring to obtain the microgel. After the completion of the polymerization, acetone is added to the microgel suspension to precipitate the microgel, followed by rinsing with acetone three times to remove the remaining monomers and the surfactant. The precipitate is filtered and then dried under reduced pressure to obtain the dried microgel in a white powder form.

Product forms of the skin cosmetic of the present invention include oil-in-water emulsion products such as emulsified foundations and sunscreen emulsions and oil-in-water cream products such as skin cream. These products can be prepared by an ordinary method using the aforementioned essential ingredients and other ingredients usually contained in skin cosmetics. Details are described below.

### "Examples 1-10 and Comparative examples 1-10"

Oil-in-water skin creams were prepared by an ordinary method using formulations shown in Table 1. The obtained skin creams (samples) were evaluated for stability and usability (spreadability on the skin, freshness, permeation, and stickiness).

### "Stability test"

The samples were left alone for one month at 50 °C and the external appearance was visually observed. The following evaluation criteria were used.

### (Evaluation criteria)

○ : No separation was observed.
Δ : Almost no separation was observed.
× : Separation occurred.

### "Evaluation (1): Absorption into the skin"

The absorption into the skin during use was evaluated with actual use testing by ten specialized panelists. The evaluation criteria are as follows:
⊚ : Eight or more specialized panelists recognized absorption into the skin during use.
○ : Six or more and less than eight specialized panelists recognized absorption into the skin during use.
Δ : Three or more and less than six specialized panelists recognized absorption into the skin during use.
× : Less than three specialized panelists recognized absorption into the skin during use.

### "Evaluation (2): Freshness"

The freshness during use was evaluated with actual use testing by ten specialized panelists. The evaluation criteria are as follows:
⊚ : Eight or more specialized panelists recognized freshness during use.
○ : Six or more and less than eight specialized panelists recognized freshness during use.
Δ : Three or more and less than six specialized panelists recognized freshness during use.
× : Less than three specialized panelists recognized freshness during use.

### "Evaluation (3): Permeating sensation on the skin"

The permeating sensation on the skin during use was evaluated with actual use testing by ten specialized panelists. The evaluation criteria are as follows:
⊚ : Eight or more specialized panelists recognized a permeating sensation on the skin during use.
○ : Six or more and less than eight specialized panelists recognized a permeating sensation on the skin during use.
△ : Three or more and less than six specialized panelists recognized a permeating sensation on the skin during use.
× : Less than three specialized panelists recognized a permeating sensation on the skin during use.

### "Evaluation (4) : Non-stickiness on the skin"

The non-stickiness on the skin during use and after use was evaluated with actual use testing by ten specialized panelists. The evaluation criteria are as follows:
⊚ : Eight or more specialized panelists recognized non-stickiness on the skin during and after use.
○ : Six or more and less than eight specialized panelists recognized non-stickiness on the skin during and after use.
△ : Three or more and less than six specialized panelists recognized non-stickiness on the skin during and after use.
× : Less than three specialized panelists reported non-stickiness on the skin during and after use.

### "Evaluation (5): Skin moisturization"

The skin moisturization one hour after use was evaluated with actual use testing by ten specialized panelists. The evaluation criteria are as follows:
⊚ : Eight or more specialized panelists recognized moisturization during and after use.
○ : Six or more and less than eight specialized panelists recognized moisturization after use.
△ : Three or more and less than six specialized panelists recognized moisturization after use.
× : Less than three specialized panelists recognized moisturization after use.

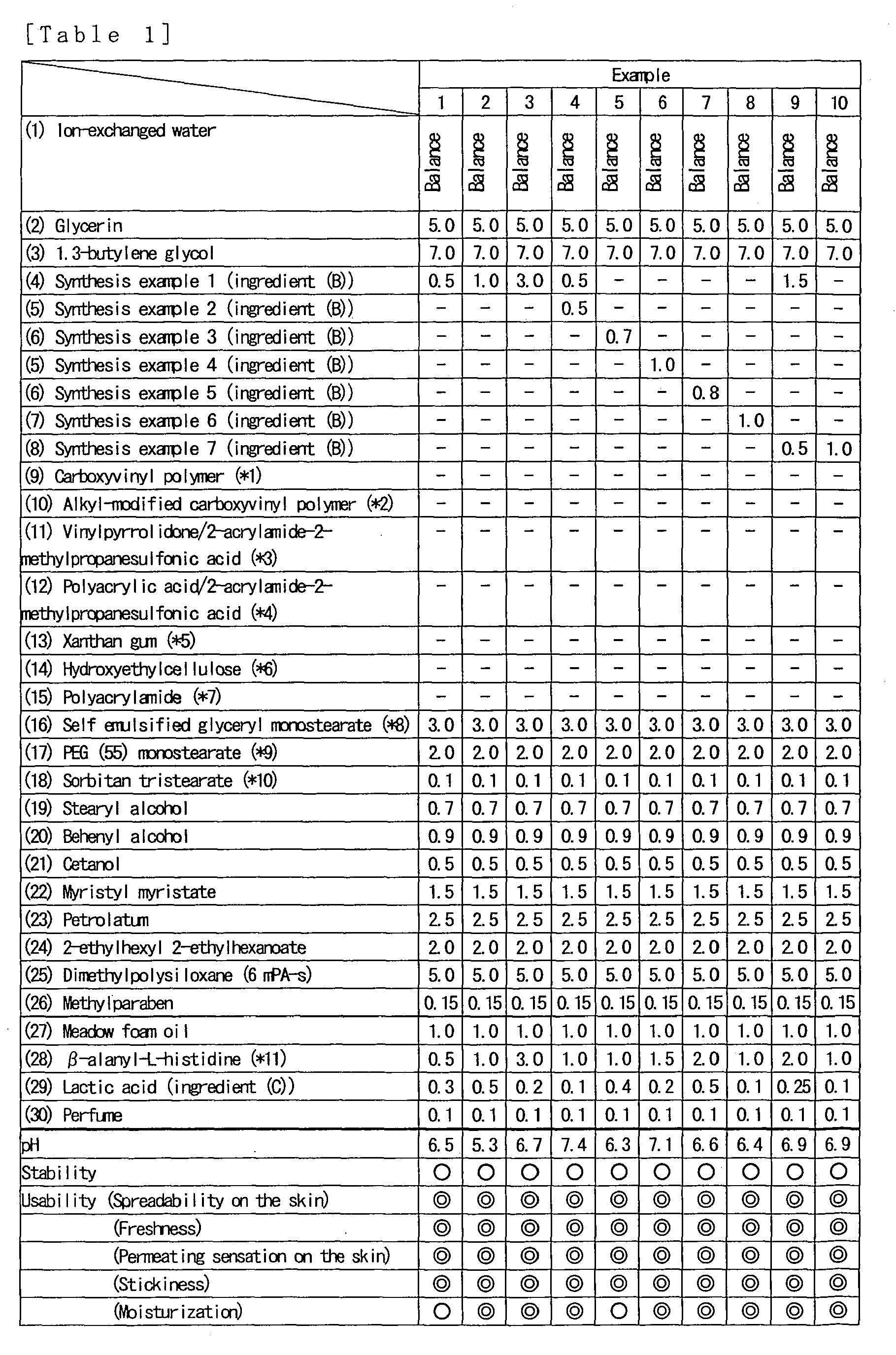

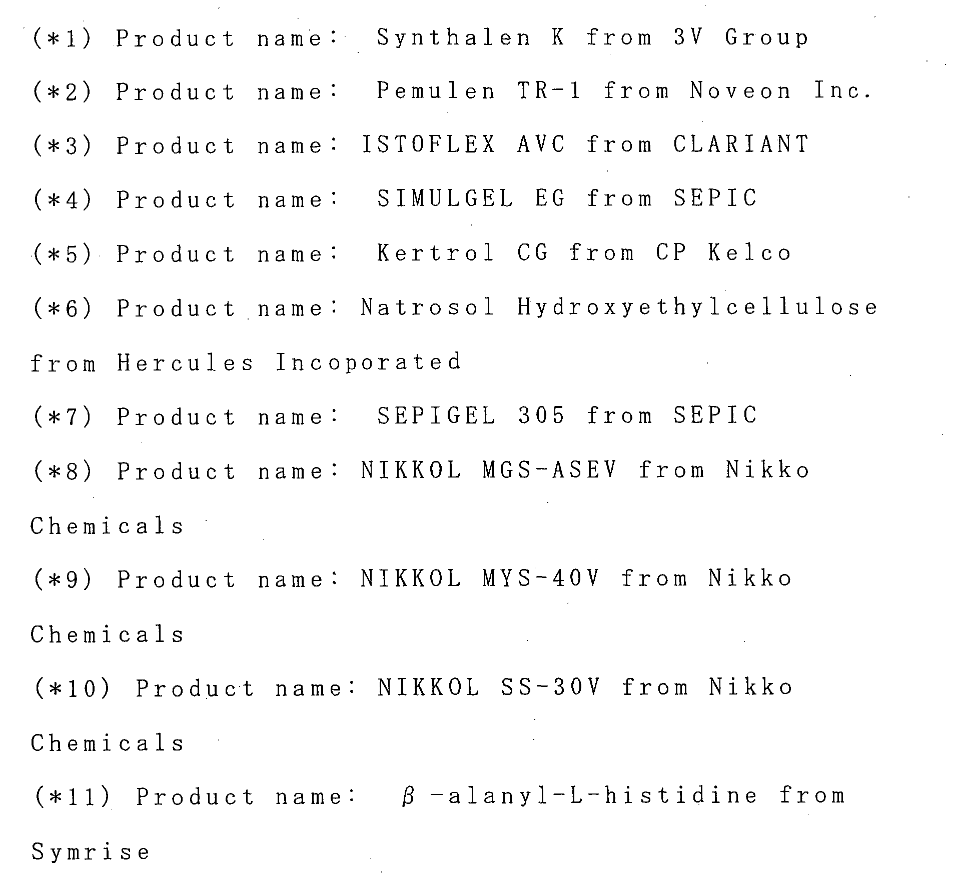

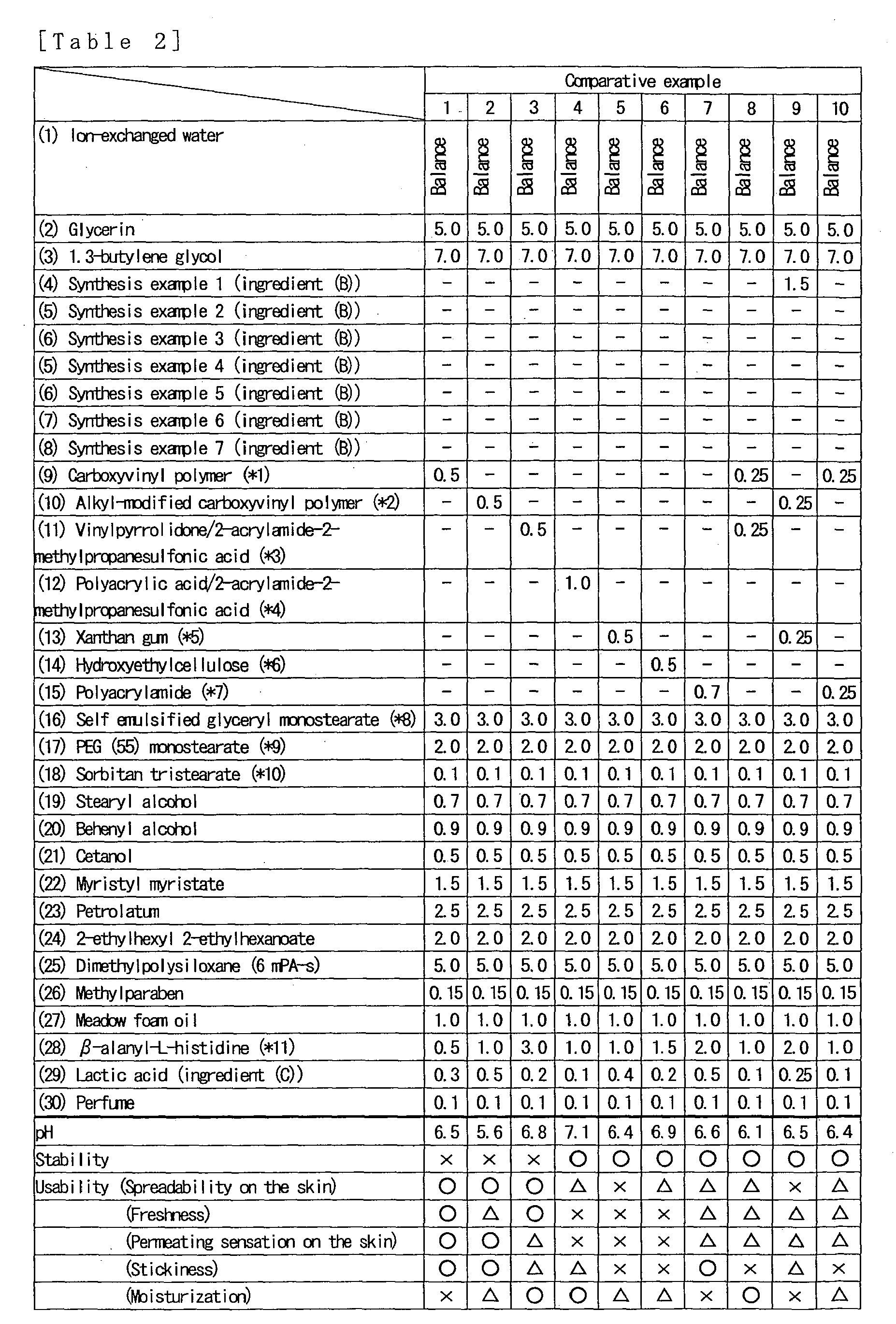

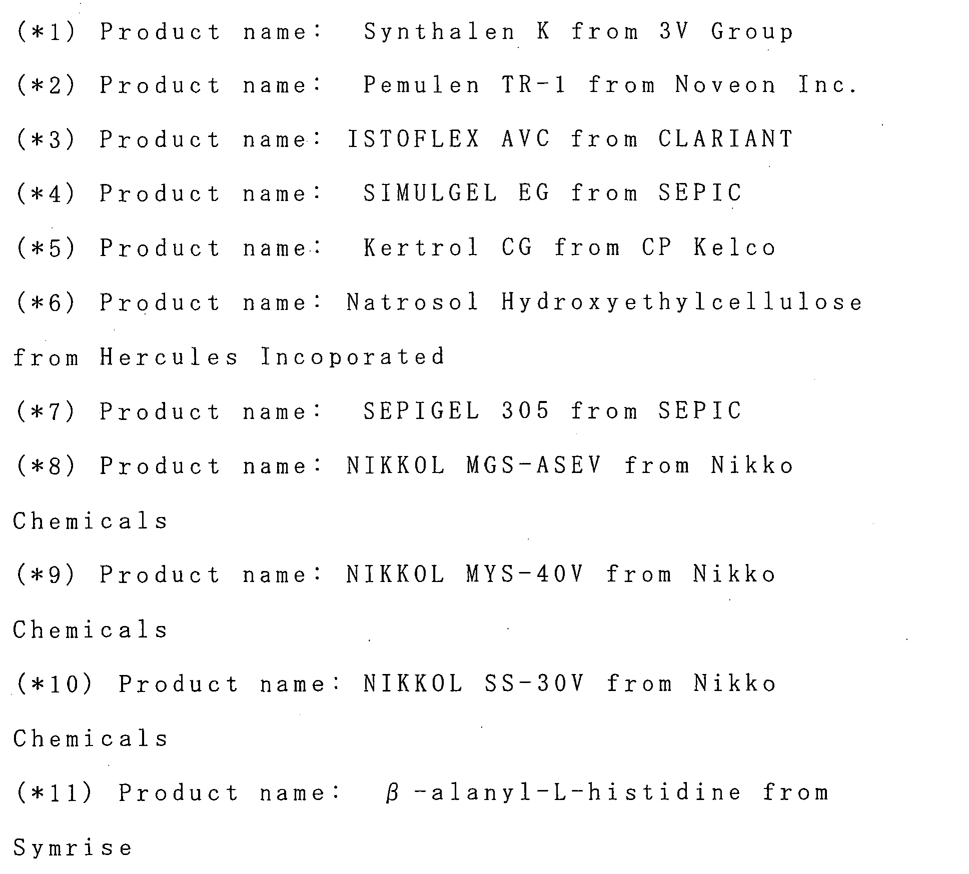

The results in Tables 1 and 2 indicate that the skin cosmetic (skin cream) of the present invention is a skin cosmetic that has superior stability, good spreadability on the skin, and also gives superior freshness, permeating sensation, and moisturizing sensation.
That is a skin cosmetic giving a good sensation during use is provided by adding β-alanyl-L-histidine and/or its salt known as a drug having effects on skin damage/UV induced immune suppression, anti-wrinkling, parakeratosis suppression, and pore reduction, combined with a specific thickener and inorganic and/or organic acid, thus adjusting the pH to a preferable range.

Other Examples of the present invention are shown below.

### Example 11: Skin cream

| A. Oil phase | |
|---|---|
| (Ingredient) | (wt%) |
| Stearic acid | 1.0 |
| Stearyl alcohol | 0.5 |
| Behenyl alcohol | 0.2 |
| Glyceryl monostearate | 2.0 |

| (Product name: Emalex GMS-F from Nihon Emulsion) | |
|---|---|
| Vitamin E acetate | 0.5 |

| Vitamin A palmitate tripropylene glycol | |
|---|---|
| dineopentanoate | 5.0 |
| Isostearyl neopentanoate | 5.0 |
| Macadamia nut oil | 1.0 |
| Tea seed oil | 1 |
| Perfume | 0.4 |
| Phenoxy ethanol | Appropriate amount |

| B. Water phase | |
|---|---|
| Microgel of Synthetic example 1 | 0.6 |
| Glycerin | 4.0 |
| 1,2-pentane diol | 3.0 |
| Sodium hyaluronate | 0.1 |
| β -alanyl-L-histidine | 1.0 |

| (Product name: Oripeptide CNS from Orient Stars LLC) | |
|---|---|
| Magnesium ascorbate phosphate | 0.1 |
| Citric acid | 0.2 |
| Trisodium edetate | 0.05 |
| Purified water | Balance |

### (Preparation method and evaluation)

Oil phase A and water phase B were each heated up to 70 °C to be dissolved completely. The A phase was added to the B phase, followed by emulsification by means of an emulsifier. The emulsion was cooled by a heat exchanger to obtain skin cream having a pH of 6.9. The obtained skin cream, in terms of sensations during use, spread well on the skin, exhibited freshness, gave a permeating sensation to the skin, and was free of stickiness.

### Example 12: Body cream

| A. Oil phase | |
|---|---|
| (Ingredient) | (wt%) |
| Cetanol | 3.0 |
| Petrolatum | 7.0 |
| Isopropyl myristate | 8.0 |
| Squalane | 10 |
| Glyceryl monostearate (self emulsified) | 2.2 |

| (Product name: NIKKOL MGS-BSEV from Nikko Chemicals) | |
|---|---|
| POE (20) sorbitan monostearate | 2.8 |
| Triethylene glycol dineopentanoate | 10.0 |
| Dipropylene glycol dineopentanoate | 10.0 |
| Vitamin E nicotinate | 2.0 |
| Perfume | 0.3 |
| γ -tocopherol | 0.05 |
| Phenoxyethanol | 0.05 |
| B. Water phase | |
| Microgel of Synthetic example 3 | 1.0 |
| 1,3-butylene glycol | 5.0 |
| Glycerin | 5.0 |
| β -alanyl-L-histidine | 1.0 |

| (Product name: Dragosine 2/060700 from Symrise) | |
|---|---|
| Dipropylene glycol | 4.0 |
| Sodium pyrrolidone carboxylate | 1.0 |
| Disodium edetate | 0.01 |
| Citric acid | 0.3 |
| Purified water | Balance |

### (Preparation method and evaluation)

Body cream having a pH of 6.7 was obtained in the same manner as in Example 11. The obtained body cream, in terms of sensations during use, spread well on the skin, exhibited freshness, gave a permeating sensation to the skin, and was free of stickiness.

### Example 13: Sunscreen emulsion

| A. Oil phase | |
|---|---|
| (Ingredient) | (wt%) |
| Behenyl alcohol | 0.5 |
| Volatile cyclic silicone | 10.0 % |
| Octocrylene | 2.0 |
| tert-butylmethoxybenzoylmethane | 2.0 |
| Tripropylene glycol dineopentanoate | 5.0 |
| Ethylhexyl methoxycinnamate | 3.0 |
| Bis ethylhexyloxyphenol methoxyphenyl triazine | |
| | 0.5 |
| Perfume | Appropriate amount |

| B. Water phase | |
|---|---|
| Purified water | Balance |
| Microgel of Synthetic example 4 | 0.3 |
| Dipropylene glycol | 7.0 |
| Decarboxy carnosine HCl | 1.5 |

| (Product name: Carcinine (2HCL) from Exsymol) | |
|---|---|
| Sodium N-stearoyl methyl taurate | 2.0 |

| (Product name: NIKKOL SMT from Nikko Chemicals) | |
|---|---|
| Lactic acid | 0.15 |

### (Preparation method and evaluation)

The oil phase and the water phase were each mixed and dissolved. Dispersion of titanium dioxide in the oil phase portion was thoroughly conducted, which was then added to the water phase portion, and emulsification was carried out using a homogenizer. The obtained sunscreen emulsion having a pH of 7.2, in terms of sensations during use, spread well on the skin, exhibited freshness, gave a permeating sensation to the skin, and was free of stickiness.

### Example 14: Emulsion

| A. Oil phase | |
|---|---|
| (Ingredient) | (wt%) |
| Squalane | 5 |
| Oleyl oleate | 3.0 |
| Petrolatum | 2.0 |
| Sorbitan sesquioleic ester | 0.8 |
| POE (20) behenyl ether | 1.2 |

| (Product name: NIKKOL BB-20 from Nikko Chemicals) | |
|---|---|
| Tripropylene glycol dineopentanoate | 2.0 |
| Isodecyl dineopentanoate | 2.0 |
| Octyldodecyl neopentanoate | 2.0 |
| Evening primrose oil | 0.5 |
| Perfume | 0.2 |
| Phenoxy ethanol | 0.2 |

| B. Water phase | |
|---|---|
| 1,3-butylene glycol | 4.5 |
| Ethanol | 3.0 |
| Microgel of Synthetic example 5 | 0.3 |
| β -alanyl-L-histidine | 2.0 |

| (Product name: Dragosine 2/060700 from Symrise) | |
|---|---|
| Citric acid | 0.3 |
| Trisodium edetate | 0.05 |
| Purified water | Balance |

### (Preparation method and evaluation)

The oil phase and the water phase were each mixed and dissolved. The water phase was added to the oil phase, followed by emulsification using a homogenizer, to obtain an emulsion having a pH of 7.1. The obtained emulsion, in terms of sensations during use, spread well on the skin, exhibited freshness, gave a permeating sensation to the skin, and was free of stickiness.

### INDUSTRIAL APPLICABILITY

The present invention provides a skin cosmetic giving a good sensation during use by adding β -alanyl-L-histidine and/or its salt known as a drug having effects on skin damage/UV induced immune suppression, anti-wrinkling, parakeratosis suppression, and pore reduction, combined with a specific thickener and inorganic and/or organic acid, thus adjusting the pH to a preferable range for skin cosmetics.

## Claims

1. A skin cosmetic comprising the following ingredients (A), (B), and (C), and also satisfying the condition (D):
(A) β -alanyl-L-histidine and/or its salt
(B) A thickener consisting of a microgel obtained by radical polymerization of water soluble ethylene-type unsaturated monomers dissolved in the dispersion phase in a composition having an organic solvent or an oil component as the dispersion medium and water as the dispersion phase.
(C) Inorganic acid and/or organic acid
(D) The pH of the skin cosmetic at 25 °C is 5.0 or higher and lower than 8.0.

2. The skin cosmetic of claim 1 wherein the microgel in said (B) is a microgel obtained by radical polymerization of a composition having an organic solvent or an oil component as the dispersion medium and water as the dispersion phase under conditions in which a single phase microemulsion or fine W/O emulsion is formed by using a surfactant.

3. The skin cosmetic of claim 1 wherein the apparent viscosity of a 0.5 wt% water dispersion of the microgel of said (B) at 25 °C is 10,000 Pa-s or more at a shear rate of 1.0 s⁻¹.

4. The skin cosmetic of claim 1 wherein the apparent viscosity of a 0.5 wt% ethanol dispersion of the microgel of said (B) at 25 °C is 5,000 Pa-s or more at a shear rate of 1.0 s⁻¹.

5. The skin cosmetic of claim 1 wherein the dynamic elastic modulus of a water or ethanol dispersion having 0.5 wt% of the microgel of said (B) at 25 °C satisfies a relationship G' (stored elastic modulus) > G" (loss elastic modulus) at a strain of 1% or less and a frequency range of 0.01-10 Hz.

6. The skin cosmetic of claims 1-5 wherein the water soluble ethylene-type unsaturated monomer of said (B) is a dialkylacrylamide represented by general formula (1) and a ionic acrylamide derivative represented by general formula (2) or (3).
General formula (1) 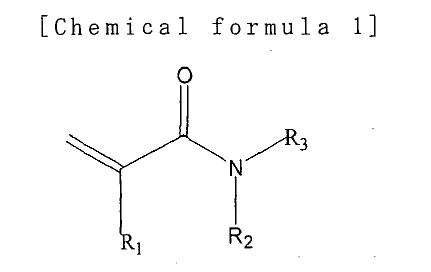 (R₁ denotes a H or methyl group; R₂ and R₃, independent of each other, denote a methyl, ethyl, propyl, or isopropyl group.)
General formula (2) 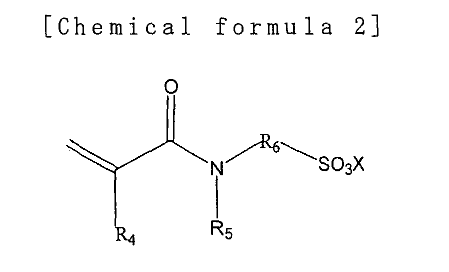 (R₄ and R₅, independent of each other, denote a H or methyl group, R₆ denotes a straight chain or branched alkyl group having 1-6 carbon atoms, and X denotes a metal ion, NH₃, or an amine compound. )
General formula (3) 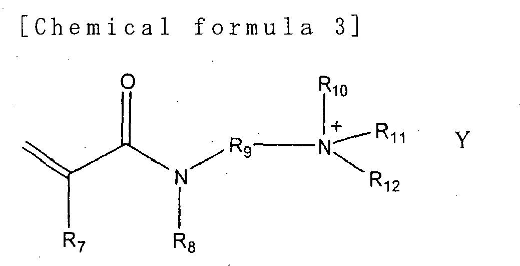 (R₇ denotes a H or methyl group, R₈ denotes a H or straight chain or branched alkyl group having 1-6 carbon atoms, R₉ denotes a straight chain or branched alkyl group having 1-6 carbon atoms, R₁₀, R₁₁, and R₁₂ denote a methyl group or ethyl group, and Y denotes an anionic counter ion.)

7. The skin cosmetic of claims 1-6 wherein the blend ratio of said ingredient (A) is 0.1-3.0 wt% of the total amount of the skin cosmetic, the blend ratio of said ingredient (B) is 0.1-5.0 wt% of the total amount of the skin cosmetic, and the blend ratio of said ingredient (C) is 0.01-1.0 wt% of the total amount of the skin cosmetic.

8. The skin cosmetic of claim 1-7 wherein the inorganic acid and/or organic acid of said ingredient (C) is one, two, or more chosen from a group consisting of phosphoric acid, lactic acid, and citric acid.
